# EUROPÄISCHE PATENTSCHRIFT

(11) **EP 3 120 827 B1**
(45) Veröffentlichungstag und Bekanntmachung des Hinweises auf die Patenterteilung: **06.02.2019**
(21) Anmeldenummer: 15177927.9
(22) Anmeldetag: 22.07.2015
(51) Int. Cl.: A61K 6/00, A61K 6/083

(54) **ADHÄSIVE DENTALWERKSTOFFE MIT STARK ACIDEN HAFTPOLYMEREN**
ADHESIVE DENTAL MATERIALS WITH STRONGLY ACIDIC ADHESIVE POLYMERS
MATIERES DENTAIRES ADHESIVE PRESENTANT DES POLYMERES ADHESIFS TRES ACIDES

(43) Veröffentlichungstag der Anmeldung: 25.01.2017
(73) Patentinhaber: Ivoclar Vivadent AG, 9494 Schaan (LI)
(72) Erfinder: Mozner, Norbert, 9495 Triesen (LI); Bock, Thorsten, 6800 Feldkirch (AT); Catel, Yohann, 9475 Rans-Sevelen (CH); Pospiech, Doris, 01309 Dresden (DE); Starke, Sandra, 06803 Bittelfeld-Wolfen (DE); Voit, Brigitte, 01187 Dresden (DE)
(74) Vertreter: Uexküll & Stolberg

(56) Entgegenhaltungen:
- EP-A1- 1 169 996
- US-A- 5 130 347
- US-B2- 8 129 444

## Beschreibung

Die vorliegende Erfindung betrifft Dentalwerkstoffe, die stark acide Haftpolymere enthalten und sich insbesondere als dentale Adhäsiven, Beschichtungsmaterialien, Füllungskomposite und Zemente eignen.

Durch radikalische Polymerisation härtbare Dentalwerkstoffe enthalten gewöhnlich eine polymerisierbare organische Matrix und einen oder mehrere Füllstoffe. Als polymerisierbare organische Matrix wird in den meisten Fällen eine Mischung von Monomeren, Initiatoren, Stabilisatoren, Pigmenten und weiteren Additiven verwendet (J. Viohl, K. Dermann, D. Quast, S. Venz, Die Chemie zahnärztlicher Füllungskunststoffe, Carl Hanser Verlag, München-Wien 1986, 21-27). Die Aushärtung derartiger Materialien kann durch thermische, redoxinitiierte oder lichtinduzierte radikalische Polymerisation erfolgen. In zunehmendem Maße werden auch acide Monomere zur Herstellung von Dentalmaterialien verwendet. Diese verleihen den Materialien selbstätzende Eigenschaften und verbessern ihre Haftung an der natürlichen Zahnsubstanz.

Als Harze werden meistens Mischungen von Dimethacrylaten eingesetzt (vgl. A. Peutzfeldt, Resin composites in dentistry: the monomer systems, Eur. J. Oral. Sci. 105 (1997) 97-116; J. W. Nicolson, H. M. Anstice, The chemistry of modern dental filling materials, J. Chem Ed. 76 (1999) 1497-1501; J. W. Stansburry, Curing dental resins and composites by photopolymerization, J. Esthet. Dent. 12 (2000) 300-308; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402).

Im Falle von Adhäsiven werden als Vernetzer oft Bisacrylamide und als Haftmonomere vor allem säuregruppenhaltige Methacrylate verwendet (N. Moszner, U. Salz, J. Zimmermann, Chemical aspects of self-etching anamel-dentin adhesives: A systematic review, Dent. Mat. 21 (2005) 895-910). Beispiele für vernetzende Dimethacrylate sind 2,2-Bis[4-(2-hydroxy-3-methacryloyloxypropyl)phenyl]propan (Bis-GMA), 1,6-Bis-[2-methacryloyloxyethoxycarbonylamino]-2,4,4-trimethylhexan (UDMA), Decandiol-1,10-dimethacrylat, Bismethacryloyloxymethyltricyclo-[5.2.1.]decan und Triethylenglycoldimethacrylat (TEGDMA).

Kommerziell in Dentalmaterialien genutzte Säuremonomere sind Methacryloyloxydecyldihydrogenphosphat (MDP), 2-Methacryloyloxyethyldihydrogenphosphat, 4-Methacryloyloxyethyltrimellithsäure, 2-[4-Dihydroxyphosphoryl)-2-oxabutyl]acrylsäureethylester (EAEPA) und 2-Acrylamido-2-methyl-1-propansulfonsäure Es ist bekannt, dass acide Gruppen die Oberfläche der Zahnhartsubstanz anätzen und aufrauen können, was zu einer mechanisch-verbesserten Substrathaftung an der Zahnhartsubstanz führt. Außerdem können Säuregruppen, die Ca²⁺-Kationen der Dentin/Schmelz-Oberfläche ionisch binden, was ebenfalls zu einer verbesserten Haftung an der Zahnhartsubstanz führt.

Neben aciden Monomeren werden regelmäßig auch organische Polymere mit aciden Gruppen zur Herstellung von Dentalmaterialien eingesetzt. Primär werden sie zur Herstellung von Glasionomerzementen verwendet. Hierbei handelt es sich um dentale Zemente, die durch eine ionische Reaktion zwischen den aciden Gruppen des Polymers und einer Ionen freisetzenden Füllstoffkomponente härten. Die EP 0 323 120 B1 offenbart Glasionomerzemente, die Polymere enthalten, die einerseits ionische Gruppen für die ionische Härtungsreaktion und andererseits lichthärtbare Gruppen aufweisen. Bei den ionischen Gruppen handelt es sich um Carboxylgruppen.

Die EP 0 796 607 B1 offenbart die Darstellung funktionalisierter schwach acider Polycarbonsäuren durch ringöffnende Methathesepolymerisation (RÖMP) einer Monomermischung aus Methacrylsäure-(5-norbornen-2-endo/exo-methyl)ester und Bicyclo-[2,2,1]hept-5-en-2,3-endo/exo-dicarbonsäurebis(tetrahydropyran-2-yl)ester und nachfolgender Abspaltung der Tetrahydropyran-Schutzgruppen. Die Polycarbonsäuren zeichnen sich durch eine hohe Haftung auf verschiedenen Substraten aus und eignen sich insbesondere zur Herstellung von Glasionomerzementen.

Die EP 0 951 896 B1 offenbart Dentalmaterialien, die saure Oligomere enthalten, die neben Methacrylatgruppen Carboxylgruppen aufweisen. Die Materialien härten einerseits durch eine Säure-Base-Reaktion zwischen saurem Oligomer und ionenfreisetzendem Füllstoff und andererseits durch radikalische Polymerisation.

Die EP 2 633 847 A2 offenbart Glasionomerzemente, die ein schwach acides Copolymer aus Acrylsäure und Itaconsäureanhydrid enthalten, das ebenfalls Carboxylgruppen aufweist.

Die US 3,872,047 offenbart dentale Primer, die eine Lösung eines Polymeren mit polaren und unpolaren Gruppen in Alkohol enthalten. Die polaren Gruppen sollen an metallhaltige Oberflächen binden. Die unpolaren Gruppen sind radikalisch polymerisierbar und können mit einem dentalen Restaurationsmaterial reagieren, das auf eine Schicht des Primers aufgebracht wird.

Der Erfindung liegt die Aufgabe zugrunde, Dentalwerkstoffe bereitzustellen, die sich durch eine hohe und langanhaltende Haftung an Zahnhartsubstanz, d.h. an Zahnschmelz und Dentin, auszeichnen. Die Dentalwerkstoffe sollen außerdem über eine hohe Lagerstabilität verfügen, die einen Transport und eine Lagerung der Dentalwerkstoffe ohne Aktivitätsverlust ermöglicht.

Die Aufgabe wird erfindungsgemäß durch Dentalwerkstoffe gelöst, die mindestens ein radikalisch polymerisierbares Oligomer oder Polymer mit stark aciden Phosphonsäuregruppen, mindestens ein radikalisch polymerisierbares, säuregruppenhaltiges Monomer und mindestens ein nicht acides Monomer enthalten. Die erfindungsgemäßen Dentalwerkstoffe können eine Mischung unterschiedlicher acider radikalisch polymerisierbarer Polymere enthalten.

Unter Oligomeren bzw. Polymeren werden Verbindungen mit einer zahlenmittleren Molmasse von vorzugsweise 1000 bis 200000 g/mol, besonders bevorzugt 10000 bis 100000 g/mol verstanden. Im Folgenden werden die Verbindungen unabhängig von der Molmasse einheitlich als acide Polymere oder Polymere bezeichnet.

Andere acide Gruppen sind Carbonsäuregruppen, Sulfonsäuregruppen, Monohydrogenphosphatgruppen und Dihydrogenphosphatgruppen. Das acide Polymer kann unterschiedliche Arten von Säuregruppen enthalten. Bevorzugt sind acide Polymere, die ausschließlich Phosphonsäuregruppen aufweisen.

Die stark aciden Gruppen können die Oberfläche der Zahnhartsubstanz anätzen und dadurch aufrauen, was zu einer mechanisch-verbesserten Haftung an der Zahnhartsubstanz führt. Außerdem können die Säuregruppen Ca²⁺-Kationen der Dentin/Schmelz-Oberfläche ionisch binden, was ebenfalls zu einer Verbesserung der Haftung führt. Im Rahmen der vorliegenden Erfindung wurde überraschenderweise gefunden, dass Polymere mit stark aciden Gruppen zu einer deutlich höheren Haftung zur Zahnhartsubstanz führen als eine entsprechenden Menge an aciden Monomeren, d.h. bei vergleichbarer Konzentration an Säuregruppen wird eine deutlich bessere Haftung der Dentalwerkstoffe an der Zahnhartsubstanz erzielt.

Die erfindungsgemäß verwendeten aciden Polymere sind radikalisch polymerisierbar, d.h. sie enthalten mindestens eine radikalisch polymerisierbare Gruppe. Bevorzugte radikalisch polymerisierbare Gruppen sind (Meth)acryl-, (Meth)acrylamid-, Vinyl- und Allylgruppen, besonders bevorzugt Methacryl- und Allylgruppen. Das acide Polymer kann unterschiedliche Arten von polymerisierbaren Gruppen enthalten. Bevorzugt sind acide Polymere mit einer Art von polymerisierbaren Gruppen. Die polymerisierbaren Gruppen der aciden Polymere bewirken, dass die Polymere bei der radikalischen Polymerisation in das Polymernetzwerk kovalent eingebunden werden.

Der Anteil an polymerisationsfähigen Gruppen im Polymer beträgt in Bezug auf die stark aciden Gruppen vorzugsweise 60 mol% betragen, besonders bevorzugt sind 5-30 mol% polymerisationsfähige Gruppen bezogen auf die vorhandenen Säuregruppen.

Besonders bevorzugt sind acide polymerisierbare Polymere mit Phosphonsäuregruppen, die zahlenmittlere Molmassen zwischen 10000 und 100000 g/mol haben und die als polymerisationsfähige Seitengruppen Methacryl- und/oder Allylgruppen aufweisen.

Die polymerisierbaren aciden Polymere können nach bekannten Synthesemethoden hergestellt werden. So lassen sich mit den Methoden der radikalischen Polymerisation oder kontrollierten radikalischen Polymerisation ausgehend von den entsprechenden Säuremonomeren oder deren Estern Homopolymerisate mit definierter Molmasse herstellen. Die Säuremonomere bzw. deren Ester können eine oder mehrere Säuregruppen bzw. Estergruppen pro Monomermolekül enthalten. Neben Estern können auch andere Säurederivate eingesetzt werden, d.h. Derivate aus denen die Säuregruppe freigesetzt werden kann. Im Fall von Monomeren, die keine freien Säuregruppen sondern Derivate davon enthalten, werden die Säuregruppen an einem Punkt nach der Polymerisation in einer geeigneten Reaktion freigesetzt, beispielsweise durch Hydrolyse.

Im Fall der Phosphonsäuregruppen eignen sich als monomere Säurederivate besonders (Meth)acrylate mit esterartig gebundenen Dialkyl- oder Disilylphosphonatgruppen. Nach der Polymerisation dieser Monomere lassen sich daraus die Phosphonsäuregruppen leicht freisetzten, im Fall der Disilylphosphonatgruppen beispielsweise durch einfache Umsetzung mit Wasser oder Methanol. Bevorzugte polymerisationsfähigen Phosphonatderivate sind z.B. 3-(Dimethoxyphosphoryl)propyl(meth)acrylat, 3-[Di(trimethylsilyl)phosphoryl]propyl(meth)acrylat, 2-(Dimethoxyphosphoryl)-ethyl(meth)acrylat, 2-[Di(trimethylsilyl)phosphoryl]ethyl-(meth)acrylat, 2-[4-(Dimethoxyphosphoryl)-2-oxa-butyl]acrylsäuremethylester und 2-[4-(Dimethoxyphosphoryl)-2-oxa-butyl]-acrylsäureethylester.

Durch den Einsatz von Monomermischungen, die neben den säuregruppenhaltigen Monomeren Comonomere enthalten, sind statistische Copolymerisate einfach zugänglich. Bevorzugte Comonomere sind Methyl-, Ethyl-, 2-Hydroxyethyl-, 2-Hydroxypropyl-, n-Butyl-, Benzyl-, Tetrahydrofurfuryl- und Isobornyl-(meth)acrylat sowie 2-Acetoacetoxyethylmethacrylat.

Durch den Einbau von Comonomeren in die Polymerkette ist eine gezielte Beeinflussung der Polymereigenschaften möglich, z.B. des Benetzungsverhaltens oder der Löslichkeit. Beispielsweise verbessert der Einbau von Comonomeren mit polaren Gruppen, wie 2-Hydroxyethylmethacrylat die Löslichkeit in Wasser oder Alkoholen, während der Einbau von Comonomeren mit unpolaren Gruppen wie n-Butylmethacrylat, Benzylmethacrylat oder Tetrahydrofurfurylmethacrylat die Löslichkeit in Aceton oder Essigester verbessert.

Mit den Methoden der kontrollierten radikalischen Polymerisation bzw. durch sequentielle anionische Polymerisation von entsprechenden Säuremonomerderivaten und nachfolgende Freisetzung der Säuregruppen sind auch säuregruppenhaltige sequentielle oder Blockcopolymere erhältlich.

Die Einführung von polymerisationsfähigen Gruppen erfolgt vorzugsweise durch Umsetzung der erhaltenen säuregruppen-haltigen Polymeren mit geeignet funktionalisierten polymerisationsfähigen Monomeren, beispielsweise mit 2-Hydroxyethyl(meth)acrylat, Allylalkohol, N-(Methyl)-N-(2-hydroxyethyl)acrylamid, N-(5-Hydroxypentyl)methacrylamid oder Glycidylmethacrylat.

Ein konkretes Beispiel ist die radikalische Homopolymerisation von 2-[4-(Dihydroxyphosphoryl)-2-oxa-butyl]-acrylsäureethylester mit Azobisisobutyronitril (AIBN) als Initiator und die nachfolgende polymeranaloger Umsetzung mit Glycidylmethacrylat (GMA) in einer Eintopfreaktion:

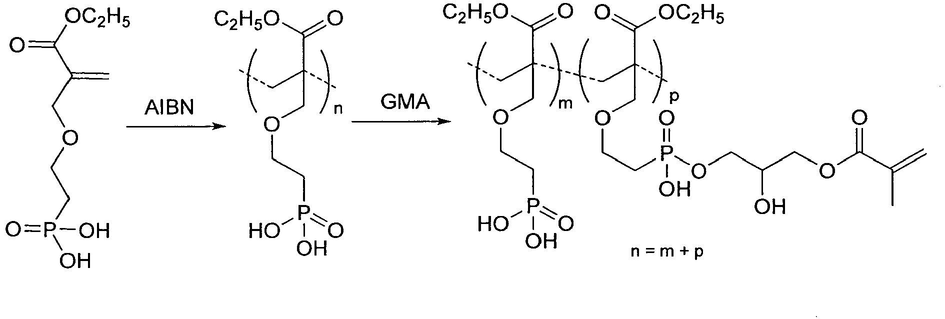

Unter einer polymeranalogen Reaktion wird eine Reaktion verstanden, bei der ein Polymer als Substrat unter Erhalt des Polymerisationsgrades, d.h. ohne Änderung der Anzahl der Monomerbausteine, umgesetzt wird, beispielsweise indem funktionelle Gruppen des Polymers zu anderen Gruppen umgesetzt werden, ohne die Polymerstruktur im Übrigen (kein Abbau und keine Vernetzung) zu verändern.

Der Anteil an Säuregruppen, der hierbei umgesetzt wird, wird vorzugsweise so gewählt, dass der Anteil an polymerisationsfähigen Gruppen im Polymer in Bezug auf die Phosphonsäuregruppen bis zu 60 mol% beträgt, besonders bevorzugt sind 5-30 mol% polymerisationsfähige Gruppen bezogen auf die vorhandenen Phosphonsäuregruppen.

Alternativ lassen sich die polymerisationsfähigen Gruppen auch durch polymeranaloge Umsetzung der Comonomerkomponente einführen. Zur Einführung der polymerisationsfähigen Gruppen durch polymeranaloge Umsetzung eignen sich besonders OH-gruppenhaltige Monomere, wie 2-Hydroxyethyl(meth)acrylat, Hydroxypropyl(meth)acrylat oder Allylalkohol, wobei die OH-Gruppen der gebildeten Polymerisate polymeranalog mit 2-Isocyanatoethylmethacrylat umsetzbar sind.

Ein konkretes Beispiel hierfür ist die radikalische Copolymerisation von 2-[4-(Dimethoxyphosphoryl)-2-oxa-butyl]-acrylsäureethylester und 2-Hydroxyethylmethacrylat (HEMA) mit AIBN als Initiator, nachfolgende polymeranaloge Umsetzung (oder partielle Umsetzung) der OH-Gruppen mit 2-Isocyanatoethylmethacrylat (IEMA), Abspaltung der Estergruppen durch Reaktion mit Trimethylsilylbromid (TMSBr) und anschließend mit Methanol:

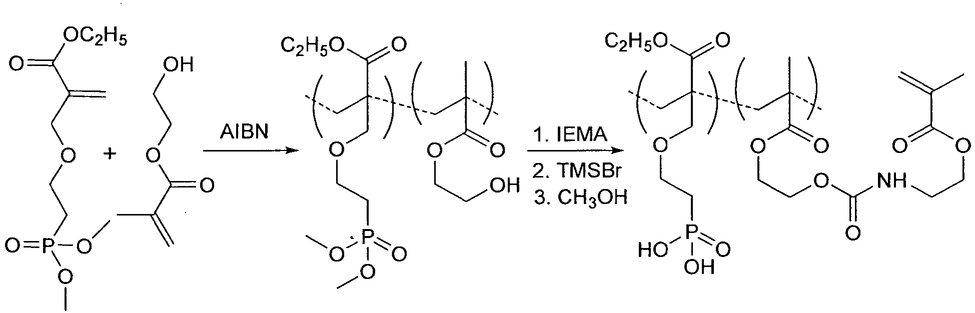

Alternativ lassen sich die erfindungsgemäßen Polymere auch so herstellen, dass zunächst ein isocyanatgruppenhaltiges Monomer wie 2-Isocyanatoethylmethacrylat radikalisch homopolymerisiert wird. Das Homopolymer wird dann mit einem hydroxlgruppenhaltigen Methacrylat, wie z.B. HEMA, und einem hydroxylgruppenhaltigen Phosphonsäuredimethylester, wie 2-Hydroxyethylphosphonsäuredimethylester polymeranalog umgesetzt, vorzugsweise mit einer Mischung aus 2-Hydroxyethylmethacrylat (HEMA) und 2-Hydroxyethylphosphonsäuredimethylester. Im dritten Schritt erfolgt dann die Freisetzung der Phosphonsäuregruppen, z.B. durch Umsetzung der Phosphonatgruppen mit TMSBr und anschliessend mit Methanol.

Bei den erfindungsgemäß verwendeten aciden Polymeren handelt es sich vorzugsweise um Poly(meth)acrylate, besonders bevorzugt um Poly(meth)acrylate, die durch radikalische Polymerisation der oben genannten aciden Monomere und ggf. Comonomere und ggf. anschließende polymeranaloge Reaktionen erhältlich sind. Die Poly(meth)acrylate weisen acide und radikalisch polymerisierbare Seitengruppen auf.

Die aciden Polymere zeichnen sich dadurch aus, dass sie einerseits eine ausreichende Anzahl an freien Säuregruppen pro Polymermolekül enthalten, um die gewünschte Substrathaftung zur Zahnhartsubstanz zu gewährleisten, und dass andererseits eine ausreichende Anzahl an polymerisierbaren Gruppen vorhanden ist, damit die aciden Polymere bei der Härtung in das Polymernetzwerk der Dentalwerkstoffe kovalent eingebunden werden können.

Die erfindungsgemäßen aciden Polymeren sind sehr gut in Alkoholen, wie z.B. Ethanol, Aceton oder in wässrigen Mischungen von Alkoholen oder von Aceton löslich. Sie sind zudem mit radikalisch polymerisierbaren Monomeren gut mischbar und zeigen eine gute radikalische Copolymerisierbarkeit. Sie verleihen Dentalwerkstoffen eine gute Haftung an der Zahnhartsubstanz, d.h. an Schmelz und Dentin, und stellen somit eine wichtige Haftkomponente dar.

Außerdem zeichnen sie sich die erfindungsgemäßen Polymere durch gute Filmbildungseigenschaften aus. Dies ist insbesondere bei der Anwendung in dentalen Adhäsiven vorteilhaft. Die Filmbildung gewährleistet eine gleichmäßige Schichtbildung des Adhäsivs beim Auftrag und verbessert die Techniktoleranz. Beispielsweise verringert eine gute Filmbildung die Gefahr, dass die flüssige Adhäsivschicht beim Verblasen des Lösungsmittels mit Pressluft beschädigt oder sogar ganz entfernt wird.

Die erfindungsgemäßen aciden Polymere eignen sich besonders zur Herstellung von adhäsiven Dentalmaterialien, wie Adhäsiven, Zementen oder Beschichtungsmaterialien.

Die erfindungsgemäßen Dentalwerkstoffe enthalten zusätzlich mindestens ein nicht acides radikalisch polymerisierbares Monomer. Es wurde gezeigt, dass die erfindungsgemäßen stark aciden Haftpolymere gut mit herkömmlichen Dentalmonomeren verträglich sind und stabile Mischungen ergeben, die bei der Polymerisation Materialien mit sehr guten adhäsiven und mechanischen Eigenschaften ergeben. Die erfindungsgemäßen Dentalwerkstoffe verfügen über eine Stabilität, die einen sicheren Transport und eine sichere Lagerung ermöglicht.

Als zusätzliche radikalisch polymerisierbare Monomere oder Mischungen radikalisch polymerisierbarer Monomere sind (Meth)acrylate bevorzugt, besonders bevorzugt Mischungen aus mono- und polyfunktionellen (Meth)acrylaten, ganz besonders bevorzugt aus mono- und difunktionellen (Meth)acrylaten. Unter Mono(meth)acrylaten werden Verbindungen mit einer, unter di- und polyfunktionellen (Meth)acrylaten Verbindungen mit zwei oder mehr, vorzugsweise 2 bis 4 radikalisch polymerisierbaren Gruppen verstanden. In allen Fällen sind Methacrylate gegenüber den Acrylaten bevorzugt.

Bevorzugte mono- oder polyfunktionelle (Meth)acrylate sind Methyl-, Ethyl-, 2-Hydroxyethyl-, 2-Hydroxypropyl-, n-Butyl-, Benzyl-, Tetrahydrofurfuryl- oder Isobornyl(meth)acrylat, 2-Acetoacetoxyethylmethacrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Bis(methacryloyloxymethyl)tricyclo[5.2.1.]decan (TCDMA), ethoxy- oder propoxyliertes Bisphenol-A-di(meth)acrylat, wie z.B. das Bisphenol-A-dimethacrylat 2-[4-(3-Methacryloyloxyethoxyethyl)-phenyl]-2-[4-(3-methacryloyloxyethyl)phenyl]-propan) (SR-348c) mit 3 Ethoxygruppen oder 2,2-Bis[4-(2-(meth)acryloxypropoxy)-phenyl]propan, Di-, Tri- oder Tetraethylenglycoldi(meth)-acrylat, Trimethylolpropantri(meth)acrylat, Pentaerythrittetra(meth)acrylat, sowie Glycerindi(meth)acrylat-Acetat, Glycerindi(meth)acrylat, 1,4-Butandioldi(meth)acrylat, 1,10-Decandioldi(meth)acrylat oder 1,12-Dodecandioldi(meth)acrylat oder Glycerintrimethacrylat (GTMA).

Weiter bevorzugt sind N-mono- oder -disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)methacrylamid sowie N-Vinylpyrrolidon oder Allylether. Diese Monomere zeichnen sich durch eine geringe Viskosität und eine hohe Hydrolysestabilität aus und eignen sich besonders als Verdünnermonomere.

Ebenfalls bevorzugt sind vernetzende Pyrrolidone, wie z.B. 1,6-Bis(3-vinyl-2-pyrrolidonyl)-hexan oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Diese Monomere zeichnen sich durch eine hohe Hydrolysestabilität aus und eignen sich besonders als Vernetzermonomere.

Besonders bevorzugte Monomere sind: CMP-1E, Bis-GMA, UDMA, TMX-UDMA, TCDMA, ethoxy- oder propoxyliertes Bisphenol-A-dimethacrylat, SR-348c, Triethylenglycoldimethacrylat, Glycerindimethacrylat, 1,10-Decandioldimethacrylat oder Glycerintrimethacrylat (GTMA) sowie N,N'-Diethyl-1,3-bis(acrylamido)-propan. Ein weiteres bevorzugtes Monomer ist Maleinsäureanhydrid.

Erfindungsgemäß wurde unerwartet gefunden, dass eine optimale Haftung an Zahnschmelz und Dentin dadurch erreicht werden kann, dass die aciden Polymere mit aciden Monomeren kombiniert werden. Die erfindungsgemäßen Dentalwerkstoffe enthalten daher auch ein oder mehrere säuregruppenhaltige radikalisch polymerisierbare Monomere (Haftmonomere; acide Monomere).

Bevorzugte säuregruppenhaltige Monomere sind polymerisationsfähige Carbonsäuren, Phosphonsäuren, Phosphorsäureester, und Sulfonsäuren.

Bevorzugte Carbonsäuren sind Maleinsäure, Acrylsäure, Methacrylsäure, 2-(Hydroxymethyl)acrylsäure, 4-(Meth)acryloyloxyethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- und -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Besonders bevorzugte Säuremonomere sind 4-(Meth)acryloyloxyethyltrimellitsäureanhydrid, 10-Methacryloyloxydecylmalonsäure, 2-Methacryloyloxyethylphosphonsäure, 2-Methacrylamidoethylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester, 2-Methacryloyloxypropylmono- oder -dihydrogenphosphat, 2-Methacryloyloxyethylmono- oder -dihydrogenphosphat, 2-Methacryloyloxyethylphenylhydrogenphosphat, 10-Methacryloyloxydecyl-dihydrogenphosphat, 6-(Methacrylamido)hexyldihydrogenphosphat und 1,3-Bis-(N-acryloyl-N-propyl-amino)-propan-2-yl-dihydrogenphosphat.

Die erfindungsgemäßen Dentalwerkstoffe enthalten vorzugsweise auch einen Initiator für die radikalische Polymerisation, besonders bevorzugt einen Photoinitiator.

Zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil bevorzugt. Besonders bevorzugt werden Campherchinon und 2,2-Dimethoxy-2-phenyl-acetophenon und besonders bevorzugt α-Diketone in Kombination mit Aminen als Reduktionsmittel, wie z.B. 4-(Dimethylamino)benzoesäureethylester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- der Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich vorteilhaft auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für die Heisshärtung sind thermische Initiatoren, wie Azoverbindungen, z.B. Azobisisobutyronitril, oder Peroxide, z.B. Dibenzoylperoxid sowie Benzpinakol und 2,2'-Dialkylbenzpinakole bevorzugt.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet.

Photopolymerisierbare Dentalwerkstoffe liegen vorzugsweise in Form einer einzigen Mischung vor, die sämtliche Bestandteile des Dentalwerkstoffs enthält. Sie enthalten als Initiator ausschließlich einen Photoinitiator und lassen durch Bestrahlung mit Licht härten.

Dualhärtende Dentalwerkstoffe enthalten neben dem Photoinitiator zusätzlich ein Peroxid, vorzugsweise Hydroperoxid als Oxidationsmittel. Dualhärtende Werkstoffe liegen vorzugsweise in Form von zwei getrennten Mischungen vor, da sonst eine vorzeitige Aushärtung erfolgen würde, wobei die erste Mischung das (Hydro)peroxid und die zweite Mischung das Thioharnstoffderivat enthält. Das Thioharnstoffderivat dient als Reduktionsmittel (Beschleuniger). Die Mischungen werden dementsprechend auch als Katalysator- und als Beschleunigerpaste bezeichnet. Eine Mischung kann in diesem Zusammenhang auch nur aus einer einzelnen Komponente oder einem einzelnen Bestandteil bestehen.

Die Härtung der dual härtenden Werkstoffe kann durch das Mischen von Katalysator- und Beschleunigerpaste ausgelöst werden. Die Zusammensetzung wird so eingestellt, dass sie nach dem Mischen der Pasten noch für einige Minuten (sog. Verarbeitungszeit) verarbeitungsfähig bleibt, nach der Verarbeitung aber schnell aushärtet. Die Verarbeitungs- und Härtungszeit lassen sich vor allem durch Art und Konzentration an (Hydro)peroxid, Thioharnstoff-Derivat und ggf. durch die Zugabe weiteren Komponenten wie Übergangsmetallredoxkatalysator und Inhibitor einstellen.

In der Regel verläuft eine durch Redox-Initiatorsysteme ausgelöste Polymerisation langsamer als eine Photopolymerisation. Dementsprechend lassen sich bei dualhärtenden Werkstoffen Überschüsse leicht entfernen, indem die durch Bestrahlung ausgelöste Photopolymerisation erst nach der Überschussentfernung erfolgt.

Weiterhin enthalten die erfindungsgemäßen Dentalwerkstoffe vorzugsweise auch mindestens einen organischen oder besonders bevorzugt anorganischen partikulären Füllstoff oder Mischungen davon. Der Füllstoff-Zusatz wird bevorzugt zur Verbesserung der mechanischen Eigenschaften und zur Anpassung der Viskosität zugegeben. Bevorzugt sind amorphe kugelförmige Materialien als Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,01-10 pm, besonders bevorzugt 0,01-1 pm, ganz besonders bevorzugt 0,2-1 pm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1000 nm). Die erfindungsgemäßen Dentalwerkstoffe enthalten aber vorzugsweise keine ionenfreisetzenden Füllstoffe, insbesondere keine Ca²⁺- oder Al³⁺-freisetzenden Gläser.

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Die erfindungsgemäßen Dentalwerkstoffe können in Abhängigkeit vom gewünschten Einsatzzweck vorzugsweise auch Lösungsmittel enthalten, insbesondere Wasser, Ethanol oder eine Mischung davon.

Gegebenenfalls können die erfindungsgemäß eingesetzten Zusammensetzungen außerdem weitere Additive enthalten, vor allem Stabilisatoren, wie z.B. Polymerisationsstabilisatoren, Aromastoffe, Farbmittel, mikrobiozide Wirkstoffe, fluoridionenabgebende Additive, optische Aufheller, Fluoreszensmittel, Weichmacher, und/oder UV-Absorber.

Erfindungsgemäß sind solche Dentalwerkstoffe besonders bevorzugt, die die folgende Zusammensetzung haben:
a) 0,1 bis 30 Gew.-%, bevorzugt 1 bis 30 Gew.-% und besonders bevorzugt 2 bis 20 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 40 Gew.-%, bevorzugt 2 bis 30 Gew.-% und besonders bevorzugt 5 bis 20 Gew.-% acide radikalisch polymerisierbare(s) Monomer(e),
d) 1 bis 80 Gew.-%, bevorzugt 1 bis 60 Gew.-% und besonders bevorzugt 5 bis 50 Gew.-% andere radikalisch polymerisierbare(s) Monomer(e), und
e) 0 bis 70 Gew.-%, bevorzugt in Abhängigkeit von der Anwendung 0 bis 20 Gew.-% (Adhäsiv) oder 10 bis 70 Gew.-% (adhäsiver Zement), besonders bevorzugt in Abhängigkeit von der Anwendung 1 bis 15 Gew.-% (Adhäsiv) oder 15 bis 60 Gew.-% (adhäsiver Zement) an Füllstoff(en).

Dentalwerkstoffe zur Verwendung als Adhäsive oder adhäsive Beschichtungsmaterialien enthalten darüber hinaus vorzugsweise zusätzlich:
f) 0 bis 70 Gew.-%, bevorzugt 0 bis 60 Gew.-% und besonders bevorzugt 0 bis 50 Gew.-% Lösungsmittel, vorzugsweise Wasser.

Dentalwerkstoffe zur Verwendung als Adhäsive oder adhäsive Beschichtungsmaterialien weisen bevorzugt folgende Zusammensetzung auf:
a) 0,1 bis 30 Gew.-%, bevorzugt 1 bis 30 Gew.-% und besonders bevorzugt 2 bis 20 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 30 Gew.-%, bevorzugt 2 bis 30 Gew.-% und besonders bevorzugt 5 bis 20 Gew.-% acide radikalisch polymerisierbare(s) Monomer(e),
d) 1 bis 80 Gew.-%, bevorzugt 1 bis 60 Gew.-% und besonders bevorzugt 5 bis 50 Gew.-% andere radikalisch polymerisierbare(s) Monomer(e),
e) 0 bis 20 Gew.-% an Füllstoff(en), und
f) 0 bis 70 Gew.-%, bevorzugt 5 bis 60 Gew.-% und besonders bevorzugt 10 bis 50 Gew.-% Lösungsmittel, vorzugsweise Wasser.

Dentalwerkstoffe zur Verwendung als adhäsive Kompositzemente oder Füllungskomposite weisen bevorzugt folgende Zusammensetzung auf:
a) 0,1 bis 30 Gew.-%, bevorzugt 0,5 bis 20 Gew.-% und besonders bevorzugt 1 bis 10 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 30 Gew.-%, bevorzugt 2 bis 20 Gew.-% und besonders bevorzugt 2 bis 15 Gew.-% acide radikalisch polymerisierbare(s) Monomer(e),
d) 1 bis 60 Gew.-%, bevorzugt 0 bis 50 Gew.-% und besonders bevorzugt 5 bis 40 Gew.-% andere radikalisch polymerisierbare(s) Monomer(e), und
e) 10 bis 70 Gew.-%, bevorzugt 20 bis 70 Gew.-% und besonders bevorzugt 40 bis 70 Gew.-% an Füllstoff(en).

Wenn nicht anders angegeben beziehen sich alle Mengenangaben auf die Gesamtmasse der Materialien. Die einzelnen Mengenbereiche können separat gewählt werden.

Besonders bevorzugt sind solche Dentalwerkstoffe, welche aus den genannten Stoffen bestehen. Weiterhin sind solche Werkstoffe bevorzugt, bei denen die einzelnen Stoffe jeweils aus den oben genannten bevorzugten und besonders bevorzugten Stoffen ausgewählt sind.

Die erfindungsgemäßen Dentalwerkstoffe eignen sich besonders als Adhäsive bzw. adhäsive Beschichtungsmaterialien, Füllungskomposite und Zemente.

Die Dentalwerkstoffe eignen sich primär zur intraoralen Anwendung durch den Zahnarzt zur Restauration geschädigter Zähne, d.h. zur therapeutischen Verwendung, z.B. als Adhäsive bzw. adhäsive Zemente, Füllungskomposite, Beschichtungs- und Verblendmaterialien (klinische Materialien). Sie können aber auch extraoral eingesetzt werden, beispielsweise bei der Herstellung oder Reparatur von Dentalrestaurationen wie Inlays, Onlays, Kronen und Brücken (technische Materialien).

Die Erfindung wird im Folgenden anhand von Ausführungsbeispielen näher erläutert.

### Ausführungsbeispiele

### Beispiel 1:

### Synthese von stark aciden Haftpolymeren mit polymerisationsfähigen Gruppen

### a) Radikalische Copolymerisation von 2-[4-(Dimethoxyphosphoryl)-2-oxa-butyl]-acrylsäureethylester (DMPAME) und 2-Hydroxyethylmethacrylat (HEMA):

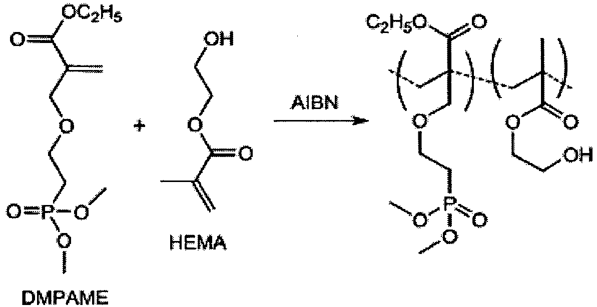

In einem ausgeheizten, mit Septum versehenen, 500 ml Schlenkkolben mit Magnetrührkern wurden 2,463 g des Initiators 2,2'-Azobis(2-methylpropionitril) (AIBN) gegeben. Nach dreimaligem Sekurieren (Evakuieren und Fluten mit Stickstoff) wurden 300 g des Lösungsmittelgemisches Ethanol/Wasser (1/1 Vol/Vol) sowie 35,137 g HEMA und 7,986 g des Phosphonsäureesters DMPAME über das Septum zugespritzt. Die Eduktkonzentration in Lösung betrug somit 15 Masse-%. Anschließend wurde das Reaktionsgemisch vollständig entgast, der Schlenkkolben mit Stickstoffgasstrom geflutet und die Polymerisation durch Eintauchen in ein vortemperiertes Wasserbad gestartet. Die Lösung wurden für 5 h bei 65 °C gerührt und das nach Reaktionsende erhaltene transparente, mäßig viskose Gemisch für 48 h in Wasser und weitere 12 h in reinem Ethanol bei Raumtemperatur dialysiert. Dafür wurde eine Dialysemembran bestehend aus regenerierter Cellulose, MW 1000 Da, verwendet. Die so gereinigte Produktlösung wurde dann bis zur Trockne eingeengt und im Vakuumtrockenschrank bei Raumtemperatur für eine Woche unter Vakuum getrocknet. Es konnte eine Ausbeute von 39,541 g (87,2 %) erzielt werden. Die Molmassenbestimmung mittels GPC ergab: Mₙ: 56000 g/mol, M_{w}: 248000 g/mol. Die Berechnung der Zusammensetzung erfolgte aus dem ¹H-NMR-Spektrum des Produktes. Die bestimmte Zusammensetzung war: 91/9 mol%/mol% (HEMA/DMPAME).

### b) Partielle polymeranaloge Umsetzung der OH-Gruppen mit 2-Isocyanatoethylmethacrylat (IEMA):

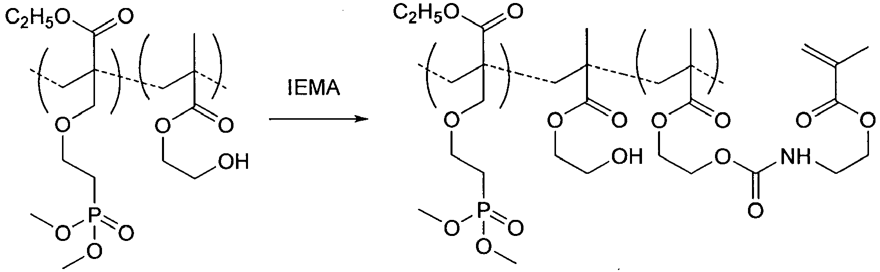

In einem ausgeheizten, mit Septum versehenen 250 ml Schlenkkolben mit Magnetrührkern wurden 5,0316 g des in Stufe a) erhaltenen Polymers sowie 0,1 g Hydrochinon eingewogen. Nach Sekurieren des Feststoffgemisches wurde unter Stickstoffatmosphäre 70 g Dimethylsulfoxid als Lösungsmittel über das Septum zugegeben. Zur Verbesserung der Löslichkeit des Gemisches wurde das Ölbad bereits zu diesem Zeitpunkt auf 50 °C temperiert. Als alle Bestandteile gelöst waren, wurden 0,221 g Dibutylzinndilaurat und 1,629 g IEMA ebenfalls vorsichtig über das Septum zugetropft. Die Eduktkonzentration in Lösung betrug somit 10 Masse-%. Das blassgelbe, transparente Reaktionsgemisch wurde für 48 h bei 50 °C unter kontinuierlichem Stickstoffgasstrom gerührt und nach Beendigung der Synthese für 120 h bei Raumtemperatur in Ethanol dialysiert. Die Trocknung der eingeengten Produktlösung erfolgte für eine Woche unter Vakuum bei Raumtemperatur. Es konnte eine Ausbeute von 5,645 g (84,7 %) erzielt werden. Die Molmassenbestimmung mittels GPC ergab: Mₙ: 65000 g/mol, M_{w}: 253000 g/mol. Die mittels ¹HNMR-Spektroskopie bestimmte Zusammensetzung war: 61/9/30 mol%/mol%/mol% (HEMA/DMPAME/IEMA-Umsetzungsprodukt).

### c) Entschützung der Phosphonsäuregruppen durch sequentielle Umsetzung mit Trimethylsilylbromid (TMSBr) und Methanol:

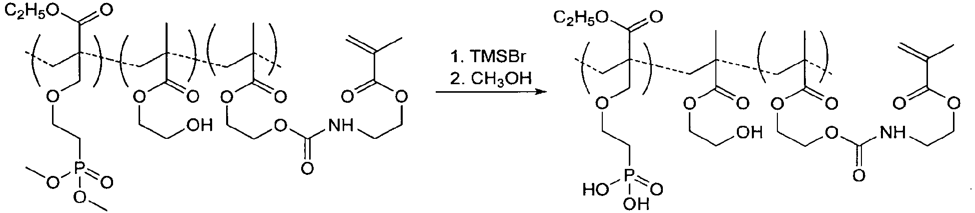

In einem mit Magnetrührkern und Septum versehenen 100 ml Schlenkkolben wurden 4,350 g des getrockneten Terpolymers aus der Vorstufe b) vorgelegt und dreimal sekuriert. Anschließend wurde unter Stickstoffatmosphäre 53 g DMF zugegeben (Ausgangskonzentration der Lösung max. 30 Masse-%). Das TMSBr (1,194 g) wurde nach vollständiger Auflösung des Polymers zugefügt und das blass-gelbe, transparente Gemisch bei 30 °C für 5 h unter Inertgas gerührt. Nach Reaktionsende wurden 55 ml Methanol zugesetzt und ebenfalls für 30 Minuten gerührt. Das leichtflüchtige Methanol und TMSBr wurden unter Zugabe von 0,02 g BHT unter Vakuum abgezogen und die verbleibende Produktlösung für 120 h in Ethanol dialysiert. Die so von DMF gereinigte Produktlösung wurde bis zur Trockne eingeengt und im Vakuumtrockenschrank bei Raumtemperatur für eine Woche unter Vakuum getrocknet. Es konnte eine Ausbeute von 4,423 g (85,4 %) an dem phosphonsäure- und methacrylatgruppenhaltigen Terpolymer erzielt werden. Das erhaltene Terpolymer zeigt eine sehr gute Löslichkeit in wässrigem Alkohol. Die Molmassenbestimmung mittels GPC ergab: Mₙ: 44500 g/mol, M_{w}: 143000 g/mol. Die Aufnahme eines ³¹P-Spektrums des Produktes ergab eine Verschiebung des Phosphorsignals von 31,52 ppm (Phosphonester) nach 23,64 ppm (Phosphonsäure). Die Berechnung der Zusammensetzung aus dem ¹H-NMR-Spektrum ergab: 61/9/30 mol%/mol%/mol% (HEMA/Phosphonsäure/IEMA-Umsetzungsprodukt).

### Beispiel 2:

### Adhäsiv auf Basis der stark aciden Haftpolymere aus Beispiel 1 und Haftuntersuchungen

Für Haftuntersuchungen wurden folgende Adhäsive hergestellt (Tabelle 1):

**Tabelle 1: Zusammensetzung der Adhäsive (Angaben in Gew.-%)**

| **Komponente** | **Adhäsiv A** | **Adhäsiv B (Vergleich)** |
|---|---|---|
| Terpolymer¹⁾ | 4.50 | - |
| HEMA | 24,50 | 25,65 |
| Bis-GMA²⁾ | 23,50 | 24,61 |
| D₃MA³⁾ | 9,00 | 9,42 |
| MDPA⁴⁾ | 5,59 | 5,85 |
| Ethanol | 13.00 | 13.61 |
| deionisiertes Wasser | 12.00 | 12.57 |
| pyrogene Kieselsäure⁵⁾ | 4,00 | 4,19 |
| Photoinitiator⁶⁾ | 3,80 | 3,98 |
| BHT | 0,11 | 0,11 |

| | | |
|---|---|---|
| ¹⁾Terpolymer aus Beispiel 1, ²⁾Bis-GMA (Additionsprodukt aus Methacrylsäure und Bisphenol-A-diglycidylether), ³⁾Decan-1,10-dioldimethacrylat (D₃MA), ⁴⁾ 10-Methacryloyloxydecyldihydrogenphosphat (MDAP), ⁵⁾Aerosil® 200 (Fa. Evonik), spez. Oberfläche 200 m²/g, ⁶⁾Mischung aus Campherchinon (A: 1,80 Gew.-% und B: 1,88 Gew.-%), 4-Dimethyl-benzoesäureethylester (A: 1,0 Gew.-% und B: 1,05 Gew.-%) und 2-(Dimethylamino)ethylmethacrylat (A: 1,0 Gew.-% und B: 1,05 Gew.-%). | | |

Zur Untersuchung der Dentinhaftung wurden Rinderzähne so in Kunststoffzylinder eingebettet, dass sich das Dentin und der Kunststoff in einer Ebene befanden. Mit einem kleinen Pinsel (Microbrush) wurde eine Schicht Adhäsiv obiger Zusammensetzung aufgepinselt, ca. 20 s das Adhäsiv auf der Zahnhartsubstanz bewegt, mit einem Luftgebläse zur Entfernung des Lösungsmittels kurz verblasen und für 10 s mit einer LED-Lampe (Bluephase, Ivoclar Vivadent) belichtet. Auf die Adhäsivschicht wurde ein Zylinder aus einem radikalisch härtenden dentalen Kompositwerkstoff (Tetric® EvoCeram; Ivoclar Vivadent AG) aufpolymerisiert. Anschließend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" bestimmt (Tabelle 2). Die Ergebnisse für das Adhäsiv A mit dem Terpolymer aus Beispiel 1 belegen im Vergleich zum polymerfreien Adhäsiv B eine signifikante Verbesserung der Haftfestigkeit auf Dentin.

**Tabelle 2: Dentin-Haftwerte**

| **Adhäsiv** | **Dentin SBS (MPa)** | **Anzahl an Säuregruppen¹⁾** |
|---|---|---|
| **A** | 33.4 ± 3.3 | 0,196 mmol/g |
| **B (Vergleich)** | 18.9 ± 0.9 | 0,182 mmol/g |

| | | |
|---|---|---|
| ¹⁾Konzentration an Säuregruppen in den Adhäsiven A und B, für das Adhäsiv A basierend auf den Säuregruppen des Terpolymers und MDPA und für das Adhäsiv B basierend auf den Säuregruppen des MDPA. | | |

## Patentansprüche

1. Radikalisch polymerisierbarer Dentalwerkstoff, der mindestens ein radikalisch polymerisierbares Oligomer oder Polymer mit stark aciden Phosphonsäuregruppen, das eine zahlenmittlere Molmasse von 1000 bis 200000 g/mol aufweist, mindestens ein radikalisch polymerisierbares, säuregruppenhaltiges Monomer und mindestens ein radikalisch polymerisierbares, nicht acides Monomer enthält.

2. Dentalwerkstoff nach Anspruch 1, bei dem das radikalisch polymerisierbare Oligomer oder Polymer eine zahlenmittlere Molmasse von 1000 bis 100000 g/mol aufweist.

3. Dentalwerkstoff nach einem der vorhergehenden Ansprüche, bei dem das radikalisch polymerisierbare Oligomer oder Polymer mindestens eine polymersationsfähige Gruppe aufweist, die aus (Meth)acryl-, (Meth)acrylamid-, Vinyl- und Allylgruppen ausgewählt ist.

4. Dentalwerkstoff nach einem der Ansprüche 1 bis 3, bei dem der Anteil an polymerisationsfähigen Gruppen im Polymer in Bezug auf die stark aciden Gruppen 60 mol% beträgt.

5. Dentalwerkstoff nach einem der Ansprüche 1 bis 4, bei dem das radikalisch polymerisierbare Oligomer oder Polymer durch radikalische Polymerisation von 3-(Dimethoxyphosphoryl)propyl(meth)acrylat, 3-[Di(trimethylsilyl)phosphoryl]propyl(meth)acrylat, 2-(Dimethoxyphosphoryl)ethyl-(meth)acrylat, 2-[Di(trimethylsilyl)phosphoryl]ethyl-(meth)acrylat, 2-[4-(Dimethoxyphosphoryl)-2-oxa-butyl]-acrylsäuremethylester und/oder 2-[4-(Dimethoxyphosphoryl)-2-oxa-butyl]acrylsäureethylester und anschließende Freisetzung der Phosphonsäureestergruppen durch Umsetzung mit Wasser oder Methanol erhältlich ist.

6. Dentalwerkstoff nach einem der Ansprüche 1 bis 5, bei dem das radikalisch polymerisierbare Oligomer oder Polymer mindestens ein Comonomer enthält, das aus Methyl-, Ethyl-, 2-Hydroxyethyl-, 2-Hydroxypropyl-, n-Butyl-, Benzyl-, Tetrahydrofurfuryl-, Isobornyl(meth)acrylat und 2-Acetoacetoxyethylmethacrylat ausgewählt ist.

7. Dentalwerkstoff nach einem der vorhergehenden Ansprüche, der als weiteres radikalisch polymerisierbares Monomer ein mono- oder polyfunktionelles (Meth)acrylat, eine Mischung aus mono- oder polyfunktionellen (Meth)acrylaten, oder eine Mischung aus mono- und difunktionellen (Meth)acrylaten enthält.

8. Dentalwerkstoff nach einem der vorhergehenden Ansprüche, der zusätzlich einen Initiator für die radikalische Polymerisation enthält, vorzugsweise einen Photoinitiator oder einen Photoinitiator in Kombination mit einem Peroxid oder Hydroperoxid.

9. Dentalwerkstoff nach einem der vorhergehenden Ansprüche, der zusätzlich mindestens einen organischen oder anorganischen partikulären Füllstoff oder eine Mischung davon enthält.

10. Dentalwerkstoff nach einem der vorhergehenden Ansprüche, der
a) 0,1 bis 30 Gew.-%, bevorzugt 1 bis 30 Gew.-% und besonders bevorzugt 2 bis 20 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 40 Gew.-%, bevorzugt 2 bis 30 Gew.-% und besonders bevorzugt 5 bis 20 Gew.-% acide radikalisch polymerisierbare(s) Monomer(e),
d) 1 bis 80 Gew.-%, bevorzugt 1 bis 60 Gew.-% und besonders bevorzugt 5 bis 50 Gew.-% radikalisch polymerisierbare(s) Monomer(e), und
e) 0 bis 70 Gew.-%, bevorzugt bis 20 Gew.-% (Adhäsiv) oder 10 bis 70 Gew.-% (adhäsiver Zement) an Füllstoff (en) und ggf.
f) 0 bis 70 Gew.-%, bevorzugt 0 bis 60 Gew.-% und besonders bevorzugt 0 bis 50 Gew.-% Lösungsmittel enthält, jeweils bezogen auf die Gesamtmasse des Werkstoffs.

11. Dentalwerkstoff nach Anspruch 10, der
a) 0,1 bis 30 Gew.-%, bevorzugt 1 bis 30 Gew.-% und besonders bevorzugt 2 bis 20 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 30 Gew.-%, bevorzugt 2 bis 30 Gew.-% und besonders bevorzugt 5 bis 20 Gew.-% acide radikalisch polymerisierbare(s) Monomer(e),
d) 1 bis 80 Gew.-%, bevorzugt 1 bis 60 Gew.-% und besonders bevorzugt 5 bis 50 Gew.-% radikalisch polymerisierbare(s) Monomer(e),
e) 0 bis 20 Gew.-% an Füllstoff(en), und
f) 0 bis 70 Gew.-%, bevorzugt 5 bis 60 Gew.-% und besonders bevorzugt 10 bis 50 Gew.-% Lösungsmittel enthält, jeweils bezogen auf die Gesamtmasse des Werkstoffs, zur Verwendung als Adhäsiv oder adhäsives Beschichtungsmaterial.

12. Dentalwerkstoff nach Anspruch 10, der
a) 0,1 bis 30 Gew.-%, bevorzugt 1 bis 30 Gew.-% und besonders bevorzugt 2 bis 20 Gew.-% mindestens eines aciden Polymers,
b) 0,01 bis 10 Gew.-%, bevorzugt 0,1 bis 3,0 Gew.-% und besonders bevorzugt 0,5 bis 3,0 Gew.-% an Initiator(en),
c) 1 bis 30 Gew.-%, bevorzugt 2 bis 20 Gew.-% und besonders bevorzugt 2 bis 15 Gew.-% acide radikalisch polymerisierbare(s) Monomer(e),
d) 0 bis 60 Gew.-%, bevorzugt 0 bis 50 Gew.-% und besonders bevorzugt 5 bis 40 Gew.-% radikalisch polymerisierbare(s) Monomer(e), und
e) 10 bis 70 Gew.-%, bevorzugt 20 bis 70 Gew.-% und besonders bevorzugt 40 bis 70 Gew.-% an Füllstoff(en) enthält, jeweils bezogen auf die Gesamtmasse des Werkstoffs, zur Verwendung als adhäsiver Kompositzement oder Füllungskomposit.

13. Dentalwerkstoff nach einem der vorhergehenden Ansprüche zur intraoralen therapeutischen Verwendung zur Restauration geschädigter Zähne, als dentales Adhäsiv, Beschichtungsmaterial, Füllungskomposit oder Zement.

14. Verwendung eines Dentalwerkstoffes nach einem der Ansprüche 1 bis 12 als Material zur extraoralen Herstellung oder Reparatur von Dentalrestaurationen.

## Claims

1. Radically polymerisable dental material which comprises at least one radically polymerisable oligomer or polymer with strongly acidic phosphonic acid groups, which has a number-average molecular weight of 1,000 to 200,000 g/mol, at least one radically polymerisable, acid group-containing monomer and at least one radically polymerisable, non-acidic monomer.

2. Dental material according to claim 1 in which the radically polymerisable oligomer or polymer has a number-average molecular weight of 1,000 to 100,000 g/mol.

3. Dental material according to one of the preceding claims in which the radically polymerisable oligomer or polymer has at least one polymerisable group that is selected from (meth)acrylic groups, (meth)acrylamide groups, vinyl and allyl groups.

4. Dental material according to one of claims 1 to 3 in which the proportion of polymerisable groups in the polymer relative to the strongly acidic groups is 60 mol %.

5. Dental material according to one of claims 1 to 4 in which the radically polymerisable oligomer or polymer is obtainable by the radical polymerisation von 3-(dimethoxyphosphoryl)propyl (meth)acrylate, 3-[di(trimethylsilyl)phosphoryl]propyl (meth)acrylate, 2-(dimethoxyphosphoryl)ethyl (meth)acrylate, 2-[di(trimethylsilyl)phosphoryl]ethyl (meth)acrylate, 2-[4-(dimethoxyphosphoryl)-2-oxa-butyl]-acrylic acid methyl ester and/or 2-[4-(dimethoxyphosphoryl)-2-oxa-butyl]acrylic acid ethyl ester, and subsequent release of the phosphonic acid ester groups by treatment with water or methanol.

6. Dental material according to one of claims 1 to 5 in which the radically polymerisable oligomer or polymer comprises at least one comonomer that is selected from methyl (meth)acrylate, ethyl (meth)acrylate, 2-hydroxyethyl (meth)acrylate, 2-hydroxypropyl (meth)acrylate, n-butyl (meth)acrylate, benzyl (meth)acrylate, tetrahydrofurfuryl (meth)acrylate, isobornyl (meth)acrylate and 2-acetoacetoxyethyl methacrylate.

7. Dental material according to one of the previous claims which comprises at least one additional radically polymerisable monomer, a mono- or polyfunctional (meth)acrylate, a mixture of mono- or polyfunctional (meth)acrylates, or a mixture of mono- and difunctional (meth)acrylates.

8. Dental material according to one of the previous claims which additionally comprises an initiator for the radical polymerisation, preferably a photoinitiator or a photoinitiator in combination with a peroxide or hydroperoxide.

9. Dental material according to one of the previous claims which additionally comprises at least one organic or inorganic particulate filler or a mixture thereof.

10. Dental material according to one of the previous claims which comprises
a) 0.1 to 30 wt %, preferably 1 to 30 wt % and particularly preferably 2 to 20 wt % of at least one acidic polymer,
b) 0.01 to 10 wt %, preferably 0.1 to 3.0 wt % and particularly preferably 0.5 to 3.0 wt % initiator(s),
c) 1 to 40 wt %, preferably 2 to 30 wt % and particularly preferably 5 to 20 wt % acidic radically polymerisable monomer(s),
d) 1 to 80 wt %, preferably 1 to 60 wt % and particularly preferably 5 to 50 wt % radically polymerisable monomer(s), and
e) 0 to 70 wt %, preferably up to 20 wt % (adhesive) or 10 to 70 wt % (adhesive cement) of filler(s) and optionally
f) 0 to 70 wt %, preferably 0 to 60 wt % and particularly preferably 0 to 50 wt % solvent,
each based on the total mass of the material.

11. Dental material according to claim 10 which comprises
a) 0.1 to 30 wt %, preferably 1 to 30 wt % and particularly preferably 2 to 20 wt % of at least one acidic polymer,
b) 0.01 to 10 wt %, preferably 0.1 to 3.0 wt % and particularly preferably 0.5 to 3.0 wt % initiator(s),
c) 1 to 30 wt %, preferably 2 to 30 wt % and particularly preferably 5 to 20 wt % of acidic radically polymerisable monomer(s),
d) 1 to 80 wt %, preferably 1 to 60 wt % and particularly preferably 5 to 50 wt % of radically polymerisable monomer(s),
e) 0 to 20 wt % of filler(s), and
f) 0 to 70 wt %, preferably 5 to 60 wt % and particularly preferably 10 to 50 wt % solvent,
each based on the total mass of the material, for use as adhesive or adhesive coating material.

12. Dental material according to claim 10 which comprises
a) 0.1 to 30 wt %, preferably 1 to 30 wt % and particularly preferably 2 to 20 wt % of at least one acidic polymer,
b) 0.01 to 10 wt %, preferably 0.1 to 3.0 wt % and particularly preferably 0.5 to 3.0 wt % initiator(s),
c) 1 to 30 wt %, preferably 2 to 20 wt % and particularly preferably 2 to 15 wt % acidic radically polymerisable monomer(s),
d) 0 to 60 wt %, preferably 0 to 50 wt % and particularly preferably 5 to 40 wt % radically polymerisable monomer(s), and
e) 10 to 70 wt %, preferably 20 to 70 wt % and particularly preferably 40 to 70 wt % filler(s),
each based on the total mass of the material, for use as adhesive composite cement or filling composite

13. Dental material according to one of the preceding claims for intraoral therapeutic use to restore damaged teeth, as a dental adhesive, coating material, filling composite or cement.

14. Use of a dental material according to one of claims 1 to 12 as a material for the extraoral manufacture or repair of dental restorations.

## Revendications

1. Matériau dentaire polymérisable par voie radicalaire, qui contient au moins un oligomère ou polymère polymérisable par voie radicalaire, comportant des groupes de type acide phosphonique fortement acides et présentant une masse molaire moyenne en nombre qui vaut de 1000 à 200 000 g/mol, au moins un monomère comportant des groupes acides et polymérisable par voie radicalaire, et au moins un monomère non acide et polymérisable par voie radicalaire.

2. Matériau dentaire conforme à la revendication 1, dans lequel l'oligomère ou polymère polymérisable par voie radicalaire présente une masse molaire moyenne en nombre de 1000 à 100 000 g/mol.

3. Matériau dentaire conforme à l'une des revendications précédentes, dans lequel l'oligomère ou polymère polymérisable par voie radicalaire comporte au moins un groupe apte à la polymérisation, choisi parmi les groupes acrylyle, méthacrylyle, acrylamido, méthacrylamido, vinyle et allyle.

4. Matériau dentaire conforme à l'une des revendications 1 à 3, dans lequel la proportion de groupes aptes à la polymérisation au sein du polymère, par rapport aux groupes fortement acides, vaut 60 % en moles.

5. Matériau dentaire conforme à l'une des revendications 1 à 4, pour lequel l'oligomère ou polymère polymérisable par voie radicalaire est accessible par polymérisation, par voie radicalaire, d'acrylate ou méthacrylate de 3-(diméthoxy-phosphoryl)-propyle, d'acrylate ou méthacrylate de 3-[di(triméthyl-silyl)-phosphoryl]-propyle, d'acrylate ou méthacrylate de 2-(diméthoxy-phosphoryl)-éthyle, d'acrylate ou méthacrylate de 2-[di(triméthyl-silyl)-phosphoryl]-éthyle, de 2-[4-(diméthoxy-phosphoryl)-2-oxa-butyl]-acrylate de méthyle et/ou de 2-[4-(diméthoxy-phosphoryl)-2-oxa-butyl]-acrylate d'éthyle, suivie de la libération immédiate des groupes phosphonate par réaction avec de l'eau ou du méthanol.

6. Matériau dentaire conforme à l'une des revendications 1 à 5, dans lequel l'oligomère ou polymère polymérisable par voie radicalaire contient au moins un co-monomère, choisi parmi les acrylates et méthacrylates de méthyle, d'éthyle, de 2-hydroxy-éthyle, de 2-hydroxypropyle, de n-butyle, de benzyle, de tétrahydrofurfuryle ou d'isobornyle et le méthacrylate de 2-(acétoacétyl-oxy)-éthyle.

7. Matériau dentaire conforme à l'une des revendications précédentes, qui contient, en tant que monomère polymérisable par voie radicalaire supplémentaire, un acrylate ou méthacrylate monofonctionnel ou polyfonctionnel, un mélange d'acrylates ou méthacrylates monofonctionnels ou polyfonctionnels, ou un mélange d'acrylates ou méthacrylates monofonctionnels et difonctionnels.

8. Matériau dentaire conforme à l'une des revendications précédentes, qui contient en outre un amorceur pour polymérisation par voie radicalaire, de préférence un photo-amorceur ou une combinaison d'un photo-amorceur et d'un peroxyde ou d'un hydroperoxyde.

9. Matériau dentaire conforme à l'une des revendications précédentes, qui contient en outre au moins une charge particulaire organique ou inorganique, ou un mélange de telles charges.

10. Matériau dentaire conforme à l'une des revendications précédentes, qui contient :
a) de 0,1 à 30 % en poids, de préférence de 1 à 30 % en poids et mieux encore de 2 à 20 % en poids d'au moins un polymère acide,
b) de 0,01 à 10 % en poids, de préférence de 0,1 à 3,0 % en poids et mieux encore de 0,5 à 3,0 % en poids d'un amorceur ou d'amorceurs,
c) de 1 à 40 % en poids, de préférence de 2 à 30 % en poids et mieux encore de 5 à 20 % en poids de monomère(s) acide(s) polymérisable(s) par voie radicalaire,
d) de 1 à 80 % en poids, de préférence de 1 à 60 % en poids et mieux encore de 5 à 50 % en poids de monomère(s) polymérisable(s) par voie radicalaire,
e) et de 0 à 70 % en poids, et de préférence jusqu'à 20 % en poids (adhésif) ou de 10 à 70 % en poids (ciment adhésif) de charge(s),
f) et en option, de 0 à 70 % en poids, de préférence de 0 à 60 % en poids et mieux encore de 0 à 50 % en poids de solvant,
chacun de ces pourcentages étant rapporté au poids total du matériau.

11. Matériau dentaire conforme à la revendication 10, qui contient
a) de 0,1 à 30 % en poids, de préférence de 1 à 30 % en poids et mieux encore de 2 à 20 % en poids d'au moins un polymère acide,
b) de 0,01 à 10 % en poids, de préférence de 0,1 à 3,0 % en poids et mieux encore de 0,5 à 3,0 % en poids d'un amorceur ou d'amorceurs,
c) de 1 à 30 % en poids, de préférence de 2 à 30 % en poids et mieux encore de 5 à 20 % en poids de monomère(s) acide(s) polymérisable(s) par voie radicalaire,
d) de 1 à 80 % en poids, de préférence de 1 à 60 % en poids et mieux encore de 5 à 50 % en poids de monomère(s) polymérisable(s) par voie radicalaire,
e) de 0 à 20 % en poids de charge(s),
f) et de 0 à 70 % en poids, de préférence de 5 à 60 % en poids et mieux encore de 10 à 50 % en poids de solvant,
chacun de ces pourcentages étant rapporté au poids total du matériau, pour utilisation en tant qu'adhésif ou matériau de revêtement adhésif.

12. Matériau dentaire conforme à la revendication 10, qui contient
a) de 0,1 à 30 % en poids, de préférence de 1 à 30 % en poids et mieux encore de 2 à 20 % en poids d'au moins un polymère acide,
b) de 0,01 à 10 % en poids, de préférence de 0,1 à 3,0 % en poids et mieux encore de 0,5 à 3,0 % en poids d'un amorceur ou d'amorceurs,
c) de 1 à 30 % en poids, de préférence de 2 à 20 % en poids et mieux encore de 2 à 15 % en poids de monomère(s) acide(s) polymérisable(s) par voie radicalaire,
d) de 0 à 60 % en poids, de préférence de 0 à 50 % en poids et mieux encore de 5 à 40 % en poids de monomère(s) polymérisable(s) par voie radicalaire,
e) et de 10 à 70 % en poids, et de préférence de 20 à 70 % en poids et mieux encore de 40 à 70 % en poids de charge(s),
chacun de ces pourcentages étant rapporté au poids total du matériau, pour utilisation en tant que ciment composite adhésif ou matériau composite de charge.

13. Matériau dentaire conforme à l'une des revendications précédentes pour utilisation thérapeutique intra-orale pour restauration de dents abimées, en tant qu'adhésif dentaire, matériau de revêtement, matériau composite de charge ou ciment.

14. Utilisation d'un matériau dentaire conforme à l'une des revendications 1 à 12, en tant que matériau pour fabrication ou réparation extra-orale de restaurations dentaires.
